# EUROPÄISCHE PATENTANMELDUNG

(11) **EP 0 691 114 A2**
(43) Veröffentlichungstag der Anmeldung: **10.01.1996**
(21) Anmeldenummer: 95110196.3
(22) Anmeldetag: 30.06.1995
(51) Int. Cl.: A61F 17/00, A61B 19/02, A45C 5/12

(54) **Notfallkoffer**

(30) Priorität: 09.07.1994 DE 9411155 U
(71) Anmelder: GOTTLIEB WEINMANN GERÄTE FÜR MEDIZIN UND ARBEITSSCHUTZ GMBH & CO., D-22525 Hamburg (DE)
(72) Erfinder: Schulz, Gerd, D-22763 Hamburg (DE)
(74) Vertreter: Liebelt, Rolf, Dipl.-Ing.

(57) **Zusammenfassung**

Bei einem Notfallkoffer aus zwei klappbar miteinander verbundenen Hartschalen 1 mit wannenförmigen Einsätzen 3 sind die Einsätze 3 durch stegförmige Trennwände 4 in Fächer 5 unterteilt. Die Trennwände 4 sind in Führungsschlitze 6 an den Seitenwänden 7 der Einsätze 3 eingeschoben. Zwischen der Trennwand 4 und den Seitenwänden 7 ist mindestens eine von einem Schnapphaken 9 und einem Rastvorsprung 10 gebildete formschlüssige Verriegelung vorgesehen.

## Beschreibung

Die Erfindung betrifft einen Notfallkoffer aus zwei klappbar miteinander verbundenen Hartschalen mit wannenförmigen Einsätzen, die durch stegförmige Trennwände, die in Führungsschlitze an den Seitenwänden der Einsätze eingeschoben sind, in Fächer unterteilt sind.

Derartige Notfallkoffer dienen zum Transport medizinischer Geräte und Instrumente, um bei Unfällen, z. B. Verkehrsunfällen, an verletzten Personen schon am Unfallort eine erste, häufig lebensrettende ärztliche Versorgung anwenden zu können. Die hierzu benötigten Gerätschaften sind in den Fächern des Notfallkoffers griffbereit angeordnet. Um die Anordnung der Fächer den Bedürfnissen des Benutzers des Notfallkoffers anpassen zu können, sind die Trennwände in die Führungsschlitze an den Seitenwänden des Einsatzes nur eingeschoben und nicht fest verankert, so daß sie sich während des Transportes des Notfallkoffers vom Boden des Einsatzes abheben, wodurch Spalte entstehen, durch welche kleine Gegenstände von einem Fach zum anderen gelangen können und folglich im Notfall nicht am vorgesehenen Platz griffbereit sind.

Aufgabe der Erfindung ist es nun, für Trennwände in Notfallkoffern eine Halterung zu schaffen, die ohne Beeinträchtigung der leichten Versetzbarkeit der Trennwände ein Verschieben oder Verrutschen dieser Wände während des Transportes des Koffers sicher unterbindet.

Diese Aufgabe wird ausgehend von einem Notfallkoffer der eingangs beschriebenen Art dadurch gelöst, daß an jeder Trennwand mindestens ein Schnapphaken ausgebildet ist, der mit einem Rastvorsprung an der Seitenwand zur Schaffung einer formschlüssigen Verriegelung zusammenwirkt.

Beim Einsetzen einer Trennwand in die Führungsschlitze greift unmittelbar vor dem Aufstoßen der Trennwand auf den Boden des Einsatzes die erfindungsgemäß von Schnapphaken und Rastvorsprüngen gebildete formschlüssige Verriegelung und hält die Trennwand unverrückbar, so daß sich kein unerwünschter Spalt zwischen Trennwand und Boden bilden kann, fest. Nach dem Lösen der Verriegelung kann die Trennwand aus dem Einsatz herausgenommen werden, um entweder die Aufteilung der Fächer zu ändern oder den Notfallkoffer, insbesondere dessen Einsatz zu reinigen, der durch den Gebrauch des Notfallkoffers am Unfallort stark verschmutzt wird. Zur Erleichterung der Reinigung des Einsatzes hat es sich zur Vermeidung von "Schmutzecken" bewährt, die Führungsschlitze an den Seitenwänden vor dem Erreichen der Bodenwand enden zu lassen. Zur Unterstützung dieser Maßnahme kann weiter der Stoßbereich zwischen Seiten- und Bodenwand abgeschrägt oder abgerundet ausgebildet sein, wobei die Trennwand der Kontur des Stoßbereiches angepaßt ist.

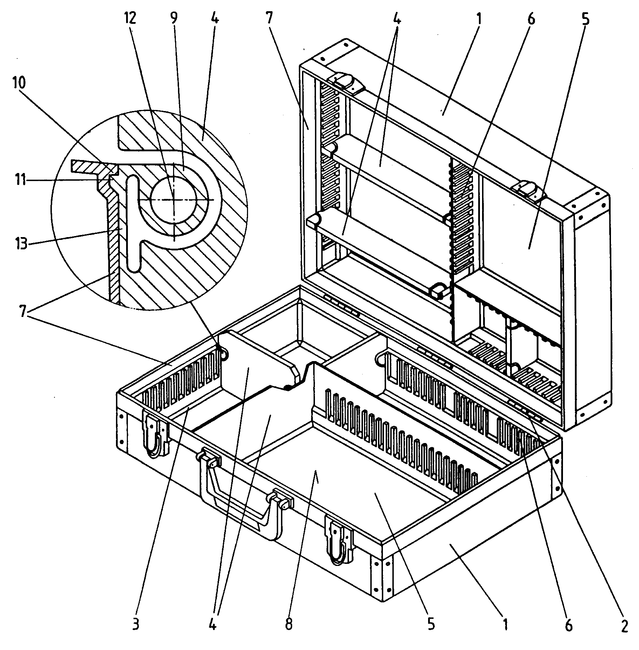

Ein Ausführungsbeispiel des erfindungsgemäßen Notfallkoffers wird noch an Hand der Zeichnung beschrieben, die einen derartigen Koffer in schematischer perspektivischer sowie die Schnappverbindung zwischen Trennwand und Einsatz in schematisch vergrößerter Ansicht zeigt.

Der dargestellte Notfallkoffer besteht aus zwei äußeren Hartschalen 1, die über Scharniere 2 klappbar miteinander verbunden sind. In die Hartschalen 1 sind wannenförmige Einsätze 3 eingefügt, die durch stegförmige Trennwände 4 in Fächer 5 unterteilt sind. Die Trennwände 4 sind in Führungsschlitze 6, die an den Seitenwänden 7 der Einsätze 3 vorgesehen sind, eingeschoben. Die gegen den Boden 8 der Einsätze 3 stoßenden Trennwände 4 sind in den Führungsschlitzen 6 mit formschlüssigen Schnappverbindungen 9, 10 unverrückbar gehalten. Diese Schnappverbindung 9, 10 besteht aus einem Schnapphaken 9, der an einem einstückig mit der Trennwand 4 ausgebildeten Steg 13 angeformt ist und mit einer Nase 11 unter einen Restvorsprung 10 im Führungsschlitz 6 greift. Zum erleichterten Lösen des Schnapphakens 9 aus der dargestellten Verriegelungsstellung, in die dieser Haken 9 federnd vorgespannt ist, dient eine in demselben 9 eingearbeitete Bohrung 12, in die z. H. mit einem Finger gegriffen werden kann, um den Schnapphaken 9 in Richtung auf die Trennwand 4 zu verstellen.

Die Erfindung ist nicht auf die dargestellte und beschriebene Ausgestaltung begrenzt, sondern schließt für den Fachmann geläufige Abwandlungen ein. So können z. B. einige der Trennwände einstückig mit dem wannenförmigen Einsatz ausgebildet sein und Führungsschlitze mit Rastvorsprüngen für versetzbare Trennwände aufweisen.

## Patentansprüche

1. Notfallkoffer aus zwei klappbar miteinander verbundenen Hartschalen mit wannenförmigen Einsätzen, die durch stegförmige Trennwände, die in Führungsschlitze an den Seitenwänden der Einsätze eingeschoben sind, in Fächer unterteilt sind, dadurch gekennzeichnet, daß an jeder Trennwand (4) mindestens ein Schnapphaken (9) ausgebildet ist, der mit einem Rastvorsprung (10) an der Seitenwand (7) zur Schaffung einer formschlüssigen Verriegelung zusammenwirkt.

2. Notfallkoffer nach Anspruch 1, dadurch gekennzeichnet, daß der Schnapphaken (9) an einem einstückig mit der Trennwand (4) ausgebildeten Steg (13) angeformt ist.

3. Notffallkoffer nach Anspruch 2, dadurch gekennzeichnet, daß an den Steg (13) eine Handhabe (12) zum Lösen des Schnapphakens (9) aus der Verriegelung mit dem Rastvorsprung (10) vorgesehen ist.

4. Notfallkoffer nach einem der vorhergehenden Ansprüche, dadurch gekennzeichnet, daß die Führungsschlitze (6) an den Seitenwänden (7) des Einsatzes (3) vor dem Erreichen der Bodenwand (8) enden.

5. Notfallkoffer nach einem der vorhergehenden Ansprüche, dadurch gekennzeichnet, daß der Stoßbereich zwischen den Seitenwänden (7) und der Bodenwand (8) des Einsatzes (3) abgeschrägt oder abgerundet gestaltet und die Trennwand (4) der Kontur des Stoßbereiches angepaßt ist.
